# EUROPEAN PATENT APPLICATION

(11) **EP 2 177 214 A1**
(43) Date of publication of application: **21.04.2010**
(21) Application number: 08166943.4
(22) Date of filing: 17.10.2008
(51) Int. Cl.: A61K 9/20, A61K 31/4709, A61P 31/04

(54) **Solid Oral Dosage Forms and Uses**

(71) Applicant: FERRER INTERNACIONAL, S.A., 08028 Barcelona (ES)
(72) Inventor: Tarrago, Cristina, 08950, Esplugues del Llobregat (ES); Santos, Benjamin, 08014, Barcelona (ES); Raga, Manuel, 08024, Barcelona (ES); Guglietta, Antonio, 08750, Molins de Rei (ES)
(74) Representative: Reitstötter - Kinzebach

(57) **Abstract**

The invention relates to newly identified uses of a desfluoroquinolone compound against various bacterial species, and new compositions and methods of use.

## Description

The present invention relates to newly identified uses of a desfluoroquinolone compound against various bacterial species, as well as new compositions and methods of use.

Desfluoroquinolone compound of formula (I) was firstly disclosed in US6335447 and equivalent patents. Its chemical name is 1-cyclopropyl-8-methyl-7-[5-methyl-6-(methylamino)-3-pyridinyl]-4-oxo-1,4-dihydro-3-quinolinecarboxylic acid, and the INN ozenoxacin has been assigned by the WHO. Its chemical formula is:

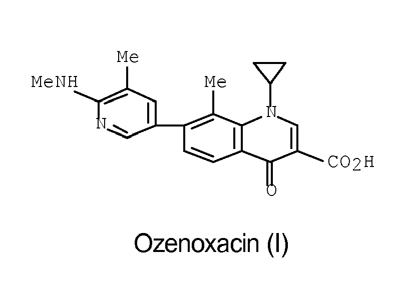

Some topical compositions comprising desfluoroquinolone compound (I) have been disclosed in JP2002356426A, JP2003226643A, EP1731138A1, WO2007015453A1 JP2007119456A, and Yamakawa, T. et al., Journal of Controlled Release (2003), 86(1), 101-103.

Thus, JP2002356426A discloses ointments and gels for the skin. An ointment comprising ozenoxacin 1%, N-methyl-2-pyrrolidone 8%, propylene glycol 14.9%, oleic acid 0.9%, diisopropanolamine 2.3%, polyethylene glycol 400 20.2%, polyethylene glycol 4000 50.2%, and water 3.2% is reported in Example 2.

JP2003226643A discloses aqueous solutions comprising ozenoxacin, cyclodextrin and a viscous agent.

EP1731138A1 discloses fine particles dispersion liquid comprising ozenoxacin to be used in the manufacture of pharmaceutical compositions.

WO2007015453A1 discloses lotions comprising ozenoxacin.

JP2007119456A discloses aqueous suspensions containing nanoparticles and solution granules of ozenoxacin to be used in the manufacture of pharmaceutical compositions. Ophthalmic solutions are preferably mentioned.

A combined use of ozenoxacin, magnesium ions, and hydroxypropyl-β-cyclodextrin specially for ophthalmic use is disclosed in Yamakawa, T. et al., Journal of Controlled Release (2003), 86(1), 101-103.

Only treatment of local infections by topical ozenoxacin compositions have been reported in the literature. No suitable ozenoxacin compositions for the treatment or prevention of severe infections have been disclosed to date. Consequently, there is a need for new compositions useful when systemic infections are involved.

Surprisingly it has been discovered that ozenoxacin is very active against a large variety of pathogenic bacteria responsible for diverse severe and systemic infections of the skin and skin structures, respiratory tract, urogenital system, gastrointestinal tract, bloodstream, bone and joints and central nervous system.

It is therefore the object of the present invention to provide the use of ozenoxacin in the manufacture of solid oral dosage forms for treating or preventing systemic bacterial infections in a mammal caused by pathogenic bacteria susceptible to ozenoxacin, wherein said solid oral dosage forms comprise an amount of 25 to 1200mg of active ingredient, and a suitable carrier.

According to the invention, the preferred amount of ozenoxacin is 50 to 800mg.

According to the invention, the suitable carrier comprises a mixture of:
(i) a water soluble inert filler selected from mannitol, xylitol, sucrose, lactose compounds, maltodextrin, dextran, dextrin, modified starches, dextrose, sorbitol, and dextrates, and mixtures thereof, in a proportion of 5 to 70% in relation to the total weight of the dosage form;
(ii) a disintegrant selected from pregelatinized starch, low-substituted hydroxypropyl cellulose, cross-linked sodium salt of a carboxymethyl ether of starch, cross-linked sodium carboxymethyl cellulose, cross-linked polyvinylpyrrolidone, sodium starch glycolate, sorbitan fatty acid esters, docusate sodium, polyoxyethylene sorbitan fatty acid esters, and microcrystalline cellulose, and mixtures thereof, in a proportion of 2 to 30% in relation to the total weight of the dosage form;
(iii) a lubricant selected from magnesium stearate, calcium stearate, glyceryl monostearate, glyceryl palmitostearate, hydrogenated castor oil, colloidal silicon dioxide, glycerin, hydrogenated vegetable oil, mineral oil, polyethylene glycol, sodium benzoate, sodium lauryl sulfate, sodium stearyl fumarate, stearic acid, talc, and zinc stearate, and mixtures thereof, in a proportion of 0.2 to 10% in relation to the total weight of the dosage form; and
(iv) a direct compression excipient selected from calcium hydrogen phosphate hydrates, spray-dried maltose, starch acetates, poly-lactic acid, and microcrystalline cellulose, and mixtures thereof, in a proportion of 5 to 70% in relation to the total weight of the dosage form.

According to the invention, the suitable carrier comprises preferably a mixture wherein:
(I) the water soluble inert filler is mannitol in a proportion of 8 to 30% in relation to the total weight of the dosage form;
(ii) the disintegrant is a mixture of pregelatinized starch in a proportion of 3 to 10% in relation to the total weight of the dosage form, and low-substituted hydroxypropyl cellulose in a proportion of 3 to 10% in relation to the total weight of the dosage form;
(iii) the lubricant is sodium stearyl fumarate in a proportion of 0.5 to 5% in relation to the total weight of the dosage form; and
(iv) the direct compression excipient is calcium hydrogen phosphate dihydrate in a proportion of 8 to 30% in relation to the total weight of the dosage form.

According to the invention, the suitable carrier comprises more preferably a mixture wherein:
(i) the proportion of mannitol is from 10 to 20% in relation to the total weight of the dosage form;
(iia) the proportion of pregelatinized starch is from 3 to 7% in relation to the total weight of the dosage form;
(iib) the proportion of low-substituted hydroxypropyl cellulose is from 3 to 7% in relation to the total weight of the dosage form;
(iii) the proportion of sodium stearyl fumarate is from 1 to 3% in relation to the total weight of the dosage form; and
(iv) the proportion of calcium hydrogen phosphate dihydrate is from 10 to 25% in relation to the total weight of the dosage form.

According to the invention, the solid oral dosage forms comprising ozenoxacin are suitable against skin and skin structure's infections caused by methicillin-susceptible *Staphylococcus aureus* (MSSA), methicillin-resistant *Staphylococcus aureus* (MRSA) including ciprofloxacin-resistant strains, methicillin-susceptible *Staphylococcus epidermidis* (MSSE), methicillin-resistant *Staphylococcus epidermidis* (MRSE), *Streptococcus pyogenes,* and Group G *Streptococci.*

According to the invention, the solid oral dosage forms comprising ozenoxacin also are suitable against respiratory tract infections caused by methicillin-susceptible *Staphylococcus aureus* (MSSA), methicillin-resistant *Staphylococcus aureus* (MRSA) including ciprofloxacin-resistant strains, penicillin-resistant *Streptococcus pneumoniae,* Beta-lactamase positive *Haemophilus influenzae,* non-typeable strains of *Haemophilus influenzae,* Beta-lactamase positive *Moraxella catarrhalis, Neisseria meningitidis, Legionella pneumophila, Mycoplasma pneumoniae, Legionella pneumophila,* and *Mycobacterium tuberculosis.*

According to the invention, the solid oral dosage forms comprising ozenoxacin also are suitable against sexually transmitted diseases and genital tract infections caused by *Streptococcus agalactiae* group B, *Neisseria gonorrhoeae, Chlamydia trachomatis, Mycoplasma hominis,* and *Ureaplasma urealyticum.*

According to the invention, the solid oral dosage forms comprising ozenoxacin also are suitable against digestive system and abdominal infections caused by methicillin-susceptible *Staphylococcus epidermidis* (MSSE), methicillin-resistant *Staphylococcus epidermidis* (MRSE), *Streptococcus pyogenes,* Group G *Streptococci, Helicobacter pylori, Bacteroides fragilis, Clostridium perfringens, Escherichia coli,* quinolone-resistant *Escherichia coli, Salmonella spp., Shigella spp.,* ciprofloxacin-susceptible *Pseudomonas aeruginosa,* and *Clostridium difficile.*

According to the invention, the solid oral dosage forms comprising ozenoxacin also are suitable against bloodstream infections caused by methicillin-susceptible *Staphylococcus aureus* (MSSA), methicillin-resistant *Staphylococcus aureus* (MRSA) including ciprofloxacin-resistant strains, methicillin-susceptible *Staphylococcus epidermidis* (MSSE), methicillin-resistant *Staphylococcus epidermidis* (MRSE), *Streptococcus pyogenes,* penicillin-resistant *Streptococcus pneumoniae,* Beta-lactamase positive *Haemophilus influenzae, Legionella pneumophila,* and *Escherichia coli.*

According to the invention, the solid oral dosage forms comprising ozenoxacin also are suitable against bone and joint infections caused by methicillin-susceptible *Staphylococcus aureus* (MSSA), methicillin-resistant *Staphylococcus aureus* (MRSA) including ciprofloxacin-resistant strains, Group G *Streptococci,* and penicillin-resistant *Streptococcus pneumoniae.*

According to the invention, the solid oral dosage forms comprising ozenoxacin also are suitable against central nervous system infections caused by methicillin-susceptible *Staphylococcus aureus* (MSSA), methicillin-resistant *Staphylococcus aureus* (MRSA) including ciprofloxacin-resistant strains, methicillin-susceptible *Staphylococcus epidermidis* (MSSE), methicillin-resistant *Staphylococcus epidermidis* (MRSE), penicillin-resistant *Streptococcus pneumoniae,* Beta-lactamase positive *Haemophilus influenzae, Listeria monocytogenes,* and *Neisseria meningitidis.*

According to the invention, the preferred amount of ozenoxacin is 50 to 800mg per tablet.

According to the invention, the suitable carrier comprises a mixture of:
(i) a water soluble inert filler selected from mannitol, xylitol, sucrose, lactose compounds, maltodextrin, dextran, dextrin, modified starches, dextrose, sorbitol, and dextrates, and mixtures thereof, in a proportion of 5 to 70% in relation to the total weight of the tablet;
(ii) a disintegrant selected from pregelatinized starch, low-substituted hydroxypropyl cellulose, cross-linked sodium salt of a carboxymethyl ether of starch, cross-linked sodium carboxymethyl cellulose, cross-linked polyvinylpyrrolidone, sodium starch glycolate, sorbitan fatty acid esters, docusate sodium, polyoxyethylene sorbitan fatty acid esters, and microcrystalline cellulose, and mixtures thereof, in a proportion of 2 to 30% in relation to the total weight of the tablet;
(iii) a lubricant selected from magnesium stearate, calcium stearate, glyceryl monostearate, glyceryl palmitostearate, hydrogenated castor oil, colloidal silicon dioxide, glycerin, hydrogenated vegetable oil, mineral oil, polyethylene glycol, sodium benzoate, sodium lauryl sulfate, sodium stearyl fumarate, stearic acid, talc, and zinc stearate, and mixtures thereof, in a proportion of 0.2 to 10% in relation to the total weight of the tablet; and
(iv) a direct compression excipient selected from calcium hydrogen phosphate hydrates, spray-dried maltose, starch acetates, poly-lactic acid, and microcrystalline cellulose, and mixtures thereof, in a proportion of 5 to 70% in relation to the total weight of the tablet.

According to the invention, the suitable carrier comprises preferably a mixture wherein:
(i) the water soluble inert filler is mannitol in a proportion of 8 to 30% in relation to the total weight of the tablet;
(ii) the disintegrant is a mixture of pregelatinized starch in a proportion of 3 to 10% in relation to the total weight of the tablet, and low-substituted hydroxypropyl cellulose in a proportion of 3 to 10% in relation to the total weight of the tablet;
(iii) the lubricant is sodium stearyl fumarate in a proportion of 0.5 to 5% in relation to the total weight of the tablet; and
(iv) the direct compression excipient is calcium hydrogen phosphate dihydrate in a proportion of 8 to 30% in relation to the total weight of the tablet.

According to the invention, the suitable carrier comprises more preferably a mixture wherein:
(i) the proportion of mannitol is from 10 to 20% in relation to the total weight of the tablet;
(iia) the proportion of pregelatinized starch is from 3 to 7% in relation to the total weight of the tablet;
(iib) the proportion of low-substituted hydroxypropyl cellulose is from 3 to 7% in relation to the total weight of the tablet;
(iii) the proportion of sodium stearyl fumarate is from 1 to 3% in relation to the total weight of the tablet; and
(iv) the proportion of calcium hydrogen phosphate dihydrate is from 10 to 25% in relation to the total weight of the tablet.

It is another object of the present invention to provide a method of treating or preventing systemic bacterial infections in a mammal caused by pathogenic bacteria susceptible to ozenoxacin by administering a tablet according to the present invention.

According to the invention, the present invention provides a method of treating or preventing skin and skin structure's infections caused by methicillin-susceptible *Staphylococcus aureus* (MSSA), methicillin-resistant *Staphylococcus aureus* (MRSA) including ciprofloxacin-resistant strains, methicillin-susceptible *Staphylococcus epidermidis* (MSSE), methicillin-resistant *Staphylococcus epidermidis* (MRSE), *Streptococcus pyogenes,* and Group G *Streptococci,* in a mammal by administering a tablet according to the present invention.

According to the invention, the present invention also provides a method of treating or preventing respiratory tract infections caused by methicillin-susceptible *Staphylococcus aureus* (MSSA), methicillin-resistant *Staphylococcus aureus* (MRSA) including ciprofloxacin-resistant strains, penicillin-resistant *Streptococcus pneumoniae,* Beta-lactamase positive *Haemophilus influenzae,* non-typeable strains of *Haemophilus influenzae,* Beta-lactamase positive *Moraxella catarrhalis, Neisseria meningitidis, Legionella pneumophila, Mycoplasma pneumoniae, Legionella pneumophila,* and *Mycobacterium tuberculosis,* in a mammal by administering a tablet according to the present invention.

According to the invention, the present invention also provides a method of treating or preventing sexually transmitted diseases and genital tract infections caused by *Streptococcus agalactiae* group B, *Neisseria gonorrhoeae, Chlamydia trachomatis, Mycoplasma hominis,* and *Ureaplasma urealyticum,* in a mammal by administering a tablet according to the present invention.

According to the invention, the present invention also provides a method of treating or preventing digestive system and abdominal infections caused by methicillin-susceptible *Staphylococcus epidermidis* (MSSE), methicillin-resistant *Staphylococcus epidermidis* (MRSE), *Streptococcus pyogenes,* Group G *Streptococci, Helicobacter pylori, Bacteroides fragilis, Clostridium perfringens, Escherichia coli,* quinolone-resistant *Escherichia coli, Salmonella spp., Shigella spp.,* ciprofloxacin-susceptible *Pseudomonas aeruginosa,* and *Clostridium difficile,* in a mammal by administering a tablet according to the present invention.

According to the invention, the present invention also provides a method of treating or preventing bloodstream infections caused by methicillin-susceptible *Staphylococcus aureus* (MSSA), methicillin-resistant *Staphylococcus aureus* (MRSA) including ciprofloxacin-resistant strains, methicillin-susceptible *Staphylococcus epidermidis* (MSSE), methicillin-resistant *Staphylococcus epidermidis* (MRSE), *Streptococcus pyogenes,* penicillin-resistant *Streptococcus pneumoniae,* Beta-lactamase positive *Haemophilus influenzae, Legionella pneumophila,* and *Escherichia coli,* in a mammal by administering a tablet according to the present invention.

According to the invention, the present invention also provides a method of treating or preventing bone and joint infections caused by methicillin-susceptible *Staphylococcus aureus* (MSSA), methicillin-resistant *Staphylococcus aureus* (MRSA) including ciprofloxacin-resistant strains, Group G *Streptococci,* and penicillin-resistant *Streptococcus pneumoniae.*

According to the invention, the present invention also provides a method of treating or preventing central nervous system infections caused by methicillin-susceptible *Staphylococcus aureus* (MSSA), methicillin-resistant *Staphylococcus aureus* (MRSA) including ciprofloxacin-resistant strains, methicillin-susceptible *Staphylococcus epidermidis* (MSSE), methicillin-resistant *Staphylococcus epidermidis* (MRSE), penicillin-resistant *Streptococcus pneumoniae,* Beta-lactamase positive *Haemophilus influenzae, Listeria monocytogenes,* and *Neisseria meningitidis,* in a mammal by administering a tablet according to the present invention.

In the present invention the term "mammal" refers to human beings. In the present invention all percentages are expressed in weight percent unless otherwise specified.

In the present invention non-limitative examples of skin and skin structure's infections caused by methicillin-susceptible *Staphylococcus aureus* (MSSA), methicillin-resistant *Staphylococcus aureus* (MRSA) including ciprofloxacin-resistant strains, methicillin-susceptible *Staphylococcus epidermidis* (MSSE), methicillin-resistant *Staphylococcus epidermidis* (MRSE), *Streptococcus pyogenes,* and Group G *Streptococci* are burn infections, carbuncle, erysipelas, folliculitis, Fournier gangrene, furunculosis, lymphogranuloma venereum, paronychia, pustule, skin abscess, yaws, impetigo, leprosy, pyoderma, and skin tuberculosis. Impetigo comprises ecthyma, impetigo bullosa, and impetigo herpetiformis. Leprosy comprises borderline leprosy, erythema nodosum leprosum, lepromatous leprosy, and tuberculoid leprosy. Pyoderma comprises pyoderma gangrenosum. Skin tuberculosis comprises lupus vulgaris.

In the present invention non-limitative examples of respiratory tract infections caused by methicillin-susceptible *Staphylococcus aureus* (MSSA), methicillin-resistant *Staphylococcus aureus* (MRSA) including ciprofloxacin-resistant strains, penicillin-resistant *Streptococcus pneumoniae,* Beta-lactamase positive *Haemophilus influenzae,* non-typeable strains of *Haemophilus influenzae,* Beta-lactamase positive *Moraxella catarrhalis, Neisseria meningitidis, Legionella pneumophila, Mycoplasma pneumoniae, Legionella pneumophila,* and *Mycobacterium tuberculosis* are diphtheria, influenza, laryngotracheobronchitis, Q fever, pertussis, tuberculous pleurisy, lung infection, infectious pneumonia, peritonsillar abscess, and nose infection. Lung infection comprises Hantavirus pulmonary syndrome, lung abscess, lung hydatid cyst, lung tuberculosis, miliary tuberculosis, and ornithosis. Infectious pneumonia comprises bacterial pneumonia, legionnaire disease, lobar pneumonia, Mycoplasma pneumonia, and Pneumocystis carinii pneumonia. Nose infection comprises chronic rhinitis, common cold, rhinopharyngitis, and rhinoscleroma.

In the present invention non-limitative examples of sexually transmitted diseases and genital tract infections caused by *Streptococcus agalactiae* group B, *Neisseria gonorrhoeae*, *Chlamydia trachomatis*, *Mycoplasma hominis,* and *Ureaplasma urealyticum* are gonococcal urethritis, gonorrhea, pyuria, trichomoniasis, urogenital tuberculosis, granuloma inguinale, gynecologic infection, ovary abscess, tuboovarian abscess, uterine tube abscess, lymphogranuloma venereum, secondary syphilis, syphilis, tabes dorsalis, ulcus molle, chlamydiasis, trachoma, and Fournier gangrene.

In the present invention non-limitative examples of digestive system infections caused by methicillin-susceptible *Staphylococcus epidermidis* (MSSE), methicillin-resistant *Staphylococcus epidermidis* (MRSE), *Streptococcus pyogenes,* Group G *Streptococci, Helicobacter pylori, Bacteroides fragilis*, *Clostridium perfringens*, *Escherichia coli,* quinolone-resistant *Escherichia coli, Salmonella spp., Shigella spp.,* ciprofloxacin-susceptible *Pseudomonas aeruginosa,* and *Clostridium difficile* are gastrointestinal infection, hepatobiliary system infection, and intestine infection. Hepatobiliary system infection comprises biliary tract infection, hepatitis, and parasitic liver disease. In his turn, biliary tract infection comprises opisthorchiasis, and parasitic liver disease comprises fascioliasis, liver amebiasis, and liver hydatid cyst. Intestine infection comprises ancylostomiasis, ascariasis, cestodiasis, diphyllobothriasis, dysentery, intestine tuberculosis, pseudomembranous colitis, salmonellosis, shigellosis, strongyloidiasis, taeniasis, tapeworm infection, and traveller diarrhea.

In the present invention non-limitative examples of abdominal infections caused by methicillin-susceptible *Staphylococcus epidermidis* (MSSE), methicillin-resistant *Staphylococcus epidermidis* (MRSE), *Streptococcus pyogenes,* Group G *Streptococci, Helicobacter pylori, Bacteroides fragilis*, *Clostridium perfringens, Escherichia coli,* quinolone-resistant *Escherichia coli, Salmonella spp., Shigella spp.,* ciprofloxacin-susceptible *Pseudomonas aeruginosa,* and *Clostridium difficile* are abdominal abscess, bacterial peritonitis, and tuberculous peritonitis.

In the present invention non-limitative examples of bone and joint infections caused by methicillin-susceptible *Staphylococcus aureus* (MSSA), methicillin-resistant *Staphylococcus aureus* (MRSA) including ciprofloxacin-resistant strains, Group G *Streptococci,* and penicillin-resistant *Streptococcus pneumoniae* are osteitis, osteomyelitis, periostitis, spondylitis including discitis, osteoarticular tuberculosis including spinal tuberculosis, and infectious arthritis.

In the present invention non-limitative examples of central nervous system infections caused by methicillin-susceptible *Staphylococcus aureus* (MSSA), methicillin-resistant *Staphylococcus aureus* (MRSA) including ciprofloxacin-resistant strains, methicillin-susceptible *Staphylococcus epidermidis* (MSSE), methicillin-resistant *Staphylococcus epidermidis* (MRSE), penicillin-resistant *Streptococcus pneumoniae,* Beta-lactamase positive *Haemophilus influenzae*, *Listeria monocytogenes,* and *Neisseria meningitidis* are coenurosis, epidural abscess, neurosyphilis, newborn tetanus, poliomyelitis, postpoliomyelitis syndrome, rabies, tetanus, brain infection, meningitis, and spinal cord infection. Non-limitative examples of brain infection are bovine spongiform encephalopathy, brain abscess, brain cystericercosis, brain malaria, progressive multifocal leukoencephalopathy, subacute sclerosing panencephalitis, subdural empyema, and tick borne encephalitis. A non-limitative example of spinal cord infection is tabes dorsalis.

Throughout the description and claims the word "comprise" and variations of the word, such as "comprising", are not intended to exclude other technical features, additives, components, or steps. Additional objects, advantages and features of the invention will become apparent to those skilled in the art upon examination of the description or may be learned by practice of the invention. The following examples are provided by way of illustration, and are not intended to be limiting of the present invention.

### EXAMPLES

### Example 1: In vitro microbiological activity against various bacterial species

### 1. Purpose

The aim of the study was to evaluate the minimum inhibitory concentration (MIC) for ozenoxacin comparatively to reference antimicrobial agents against various bacterial species.

### 2. Materials and methods

### 2.1. Antimicrobial

Reference antimicrobial compounds were selected from fluoroquinolones such as ciprofloxacin, levofloxacin, and moxifloxacin, penicillins such as amoxicillin-clavulanate (augmentin), macrolides such as clarithromycin, cephalosporins such as ceftriaxone, tetracyclines such as tigecycline, oxazolidinones such as linezolid, and polypeptide antibiotic agents such as daptomycin.

The antibacterial containing solutions were prepared as follows.

### 2.1.1 Ozenoxacin

An ozenoxacin stock solution of 1280mg/L was prepared by mixing the powder into sterile distilled water (10% of final volume) to create a suspension to which 1 N NaOH was then added (8% of final volume). This was shaken until the powder was visibly dissolved and then made up to the final required volume in sterile distilled water. Further dilutions were made in sterile distilled water and the specific test medium used for MIC determinations, as required.

### 2.1.2 Ciprofloxacin

A ciprofloxacin stock solution of 640mg/L was prepared in sterile distilled water. Further dilutions were made in sterile distilled water and the specific test medium used for MIC determinations, as required.

### 2.1.3 Levofloxacin

A levofloxacin stock solution of 640mg/L was prepared in sterile distilled water. Further dilutions were made in sterile distilled water and the specific test medium used for MIC determinations, as required.

### 2.1.4 Moxifloxacin

A moxifloxacin stock solution of 640mg/L was prepared in sterile distilled water. Further dilutions were made in sterile distilled water and the specific test medium used for MIC determinations, as required.

### 2.1.5 Amoxicillin

An amoxicillin stock solution of 1280mg/L was prepared in 0.05M phosphate buffer pH7.5 and then further diluted in sterile distilled water and test medium, as required.

### 2.1.6 Clavulanic acid

A clavulanic acid stock solution of 640mg/L was prepared in sterile distilled water. Further dilutions were made in sterile distilled water and the specific test medium used for MIC determinations, as required. To test amoxicillin-clavulanate, amoxicillin and clavulanic acid were mixed in proportions 2:1 respectively.

### 2.1.7 Clarithromycin

A clarithromycin stock solution of 640 mg/L was prepared in methanol and then further diluted in sterile distilled water and test medium, as required.

### 2.1.8 Ceftriaxone

A ceftriaxone stock solution of 640mg/L was prepared in sterile distilled water. Further dilutions were made in sterile distilled water and the specific test medium used for MIC determinations, as required.

### 2.1.9 Tigecycline

A tigecycline stock solution of 640mg/L was prepared in sterile distilled water. Further dilutions were made in sterile distilled water and the specific test medium used for MIC determinations, as required.

### 2.1.10 Liaezolid

A linezolid stock solution of 1280mg/L was prepared in sterile distilled water. Further dilutions were made in sterile distilled water and the specific test medium used for MIC determinations, as required.

### 2.1.11 Daptomycia

A daptomycin stock solution of 1280mg/L was prepared in sterile distilled water. Further dilutions were made in sterile distilled water and the specific test medium used for MIC determinations, as required. For daptomycin MIC determination all media were supplemented with Ca⁺⁺ to 50mg/L.

### 2.2 Bacterial isolates

The following bacterial isolates were tested:
1. 100 *Staphylococcus aureus* clinical isolates from blood infection, skin and soft tissue infections (SSTI) or respiratory tract infections (RTI), collected from centres worldwide during 2005-6. Equal numbers of methicillin-resistant and methicillin-susceptible *S. aureus* (MRSA and MSSA) were included;
2. 100 *Staphylococcus epidermidis* clinical isolates from blood infection, SSTI or RTI, collected from centres worldwide during 2005-6. Equal numbers of methicillin-resistant and methicillin-susceptible *S. epidermidis* (MRSE and MSSE) were included;
3. 50 Group A *Streptococci* clinical isolates from blood infection or SSTI, collected from European centres during 2004-2005;
4. 20 Group B *Streptococci* clinical isolates from blood infection or SSTI, collected from European centres during 2004-2005;
5. 20 Group C *Streptococci* clinical isolates from SSTI, collected from European centres during 2004-2005;
6. 20 Group G *Streptococci* clinical isolates from blood infection or SSTI, collected from European centres during 2004-2005;
7. 10 *Streptococcus constellatus* clinical isolates from blood infection, collected from European centres during 2004-2005;
8. 10 *Streptococcus bovis* clinical isolates from blood infection, collected from European centres during 2004-2005;
9. 10 *Streptococcus mitis* clinical isolates from blood infection, collected from European centres during 2004-2005;
10. 10 *Streptococcus sanguis* clinical isolates from blood infection, collected from European centres during 2004-2005;
11. 10 *Streptococcus oralis* clinical isolates from blood infection, collected from European centres during 2004-2005;
12. 60 *Streptococcus pneumoniae* clinical isolates from blood infection, SSTI or RTI, collected from centres worldwide during 2005-6. Equal quantities of penicillin G-susceptible, intermediate and resistant strains were included;
13. 20 library strains of *Listeria monocytogenes;*
14. 50 *Escherichia coli* clinical isolates from blood infection or SSTI, collected from centres worldwide during 2005-6;
15. 50 *Pseudomonas aeruginosa* clinical isolates from blood infection, SSTI or RTI collected from centres worldwide during 2005-6;
16. 20 library strains of *Salmonella spp*.;
17. 20 library strains of *Shigella spp.;*
18. 70 *Haemophilus influenzae* clinical isolates from RTI, collected from centres worldwide during 2005-6. 25 Beta-lactamase positive, 25 Beta-lactamase negative and 20 non-typeable strains were included;
19. 50 *Moraxella catarrhalis* clinical isolates from RTI collected from UK centres during 2005-6. Equal numbers of Beta-lactamase positive and negative strains were included;
20. 20 library strains of *Neisseria gonorrhoeae*;
21. 20 library strains of *Neisseria meningitidis;*
22. 5 library strains of *Helicobacter pylori*;
23. 20 library strains of *Bacteroides fragilis*; and
24. 10 library strains of *Clostridium difficile*.

### 2.3. MIC determinations

a) *N. gonorrhoeae*, *H. pylori* and anaerobic bacteria MIC determinations were carried out using Clinical and Laboratory Standards Institute (CLSI, formerly NCCLS) agar dilution methodology:
   - NCCLS (2004) Methods for Antimicrobial Susceptibility Testing of Anaerobic Bacteria; Approved Standard, Sixth Edition. NCCLS Document M11-A6. NCCLS, Wayne, Pennsylvania 19087-1898, USA CLSI (2006) Methods for Dilution Antimicrobial Susceptibility Tests for Bacteria that Grow Aerobically; Approved Standard, Seventh Edition. CLSI Document M7-A7. CLSI, Wayne, Pennsylvania 19087-1898, USA.
b) All other bacteria
   - MIC determinations were carried out using Clinical and Laboratory Standards Institute (CLSI, formerly NCCLS) broth microdilution methodology:

   - CLSI (2006) Methods for Dilution Antimicrobial Susceptibility Tests for Bacteria that Grow Aerobically; Approved Standard, Seventh Edition. CLSI Document M7-A7. CLSI, Wayne, Pennsylvania 19087-1898, USA.
   - CLSI (2006) Methods for Antimicrobial Dilution and Disk Susceptibility Testing of Infrequently Isolated or Fastidious Bacteria; Approved Guideline. CLSI Document M45-A. CLSI, Wayne, Pennsylvania 19087-1898, USA.

### 3. Results

### 3.1 Activity against methicillin-resistant Staphylococcus aureus (MRSA)

Summary data for ozenoxacin and comparators against 50 recent clinical strains of *Staphylococcus aureus* are shown in Table 1.

Against all fluoroquinolone-susceptible strains of MRSA, ozenoxacin was more active than all other comparator compounds with MIC as low as 0.002mg/L. Against fluoroquinolone non-susceptible strains (45 out of 50), ozenoxacin MICs were comparable to those of daptomycin and tigecycline but had increased activity compared to all other compounds including sixty-four times more activity than ciprofloxacin and levofloxacin, and thirty-two times increased activity compared to moxifloxacin. Table 1 shows ozenoxacin having a higher maximum MIC than other compounds against MRSA. This is due to 1 strain of MRSA which has MIC values greater than the highest concentration tested for all antibiotics apart from linezolid, tigecycline or daptomycin. Ozenoxacin was tested at a higher concentration range than other antibiotics and therefore produces this artifactual highest maximum value.

**Table 1. Summary MIC data for ozenoxacin and comparators against 50 methicillin-resistant Staphylococcus aureus (MRSA)**

| Antimicrobial | MIC mg/L | | | |
|---|---|---|---|---|
| | MIN | 50% | 90% | MAX |
| Amoxicillin | 8 | ≥64 | ≥64 | ≥64 |
| Augmentin | 4 | 8 | ≥64 | ≥64 |
| Ceftriaxone | 16 | ≥64 | ≥64 | ≥64 |
| Ciprofloxacin | 0.5 | ≥64 | ≥64 | ≥64 |
| Clarithromycin | 0.12 | ≥64 | ≥64 | ≥64 |
| Daptomycin | 0.12 | 0.25 | 0.5 | 1 |
| Ozenoxacin | 0.002 | 0.12 | 1 | ≥128 |
| Levofloxacin | 0.25 | 16 | ≥64 | ≥64 |
| Linezolid | 1 | 2 | 2 | 4 |
| Moxifloxacin | 0.06 | 4 | 32 | ≥64 |
| Tigecycline | 0.06 | 0.25 | 0.25 | 1 |

### 3.2 Activity against methicillin-susceptible Staphylococcus aureus (MSSA)

Summary data for ozenoxacin and comparators against 50 recent clinical strains of *Staphylococcus aureus* are shown in Table 2.

Against all fluoroquinolone-susceptible strains of MSSA, ozenoxacin was more active than all other comparator compounds with MIC as low as ≤0.0005mg/L. Against fluoroquinolone non-susceptible strains (4 out of 38), ozenoxacin MICs were comparable to those of daptomycin and tigecycline but had increased activity compared to all other compounds including over one hundred times more activity than ciprofloxacin, sixty-four times more activity than levofloxacin and sixteen times more activity than moxifloxacin.

**Table 2: Summary MIC data for ozenoxacin and comparators against 50 methicillin-susceptible Staphylococcus aureus (MSSA)**

| Antimicrobial | MIC mg/L | | | |
|---|---|---|---|---|
| | MIN | 50% | 90% | MAX |
| Amoxicillin | 0.12 | 2 | 16 | ≥64 |
| Augments | 0.12 | 0.5 | 1 | 2 |
| Ceftriaxone | 0.5 | 4 | 8 | 8 |
| Ciprofloxacin | 0.12 | 0.5 | 1 | ≥64 |
| Clarithromycin | ≤0.03 | 0.25 | ≥64 | ≥64 |
| Daptomycin | 0.06 | 0.25 | 0.25 | 0.5 |
| Ozenoxacin | ≤0,0005 | 0.002 | 0.008 | 0.5 |
| Levofloxacin | 0.06 | 0.25 | 0.5 | ≥64 |
| Linezolid | 1 | 2 | 2 | 4 |
| Moxifloxacin | ≤0.015 | 0.06 | 0.12 | 16 |
| Tigecycline | 0.06 | 0.12 | 0.25 | 0.5 |

### 3.3 Activity against methicillin-resistant Staphylococcus epidermidis (MRSE)

Summary data for ozenoxacin and comparators against 49 recent clinical strains of *Staphylococcus epidermidis* are shown in Table 3.

Against fluoroquinolone-susceptible strains of MRSE, ozenoxacin had greatly increased activity compared to all other compounds, where MICs were as low as 0.002mg/L in some instances. Against fluoroquinolone-resistant strains (35 out of 50), ozenoxacin MICs were comparable to those of daptomycin, linezolid and tigecycline. There was increased activity compared to all other compounds including ciprofloxacin, levofloxacin or moxifloxacin where ozenoxacin was sixty-four, sixty-four and thirty-two times more active respectively.

**Table 3. Summary MIC data for ozenoxacin and comparators against 49 methicillin-resistant Staphylococcus epidermidis (MRSE)**

| Antimicrobial | MIC **mg/L** | | | |
|---|---|---|---|---|
| | MIN | 50% | 90% | MAX |
| Amoxicillin | 0.03 | 4 | 32 | 32 |
| Augmentin | 0.03 | 1 | 8 | 16 |
| Ceftriaxone | 2 | 16 | ≥64 | ≥64 |
| Ciprofloxacin | 0.12 | 4 | ≥64 | ≥64 |
| Clarithromycin | ≤0.03 | ≥64 | ≥64 | ≥64 |
| Daptomycin | 0.06 | 0,25 | 0.5 | 0.5 |
| Ozenoxacin | 0.002 | 0.06 | 1 | 2 |
| Levofloxacin | 0.12 | 4 | ≥64 | ≥64 |
| Linezolid | 0.5 | 0.5 | 1 | 2 |
| Moxifloxacin | 0.03 | 1 | 32 | ≥64 |
| Tigecycline | 0.015 | 0.12 | 0.5 | 0.5 |

### 3.4 Activity against methicillin-susceptible Staphylococcus epidermidis (MSSE)

Summary data for ozenoxacin and comparators against 50 recent clinical strains of *Staphylococcus epidermidis* are shown in Table 4.

Against fluoroquinolone-susceptible strains of MSSE, ozenoxacin had greatly increased activity compared to all other compounds, where MIC were as low as 0.001 mg/L and activity was over 1000 times increased in some instances (clarithromycin). Against fluoroquinolone-resistant strains (7 out of 49), ozenoxacin MICs were comparable to those of daptomycin, linezolid and tigecycline but there was increased activity compared to all other compounds including sixty-four times more activity than ciprofloxacin and levofloxacin, and sixteen times increased activity compared to moxifloxacin.

**Table 4. Summary MIC data for ozenoxacin and comparators against 50 methicillin-susceptible Staphylococcus epidermidis (MSSE)**

| Antimicrobial | MIC mg/l | | | |
|---|---|---|---|---|
| | MIN | 50% | 90% | MAX |
| Amoxicillin | 0.03 | 0.25 | 0.5 | 2 |
| Augrnetitin | 0.03 | 0.12 | 0.25 | 0.5 |
| Ceftnaxone | 0.5 | 2 | 4 | 4 |
| Ciprofloxacin | 0.06 | 0.12 | 4 | ≥64 |
| Clarithromycin | ≤0.03 | 0.12 | ≥64 | ≥64 |
| Daptomycin | 0,06 | 0.25 | 0.5 | 1 |
| Ozenoxacin | 0.001 | 0.002 | 0.06 | 0.12 |
| Levofloxacin | 0.06 | 0.12 | 4 | 16 |
| Linezolid | 0.25 | 0.5 | 1 | 1 |
| Moxifloxacín | ≤0.015 | 0.06 | 1 | 4 |
| Tigecycline | 0.03 | 0.12 | 0.25 | 0.5 |

### 3.5 Activity against Group A Streptococci

Summary data for ozenoxacin and comparators against 50 recent clinical strains of Group A *Streptococci* are shown in Table 5.

The table shows that ozenoxacin was sixty-four times more active than ciprofloxacin or levofloxacin, eight times more active than moxifloxacin, thirty-two times more active than linezolid and twice as active as tigecycline. These data show ozenoxacin to be highly active against clarithromycin non-susceptible strains also.

**Table 5. Summary MIC data for ozenoxacin and comparators against 50 Group A Streptococci**

| Antimicrobial | MIC mg/L | | | |
|---|---|---|---|---|
| | MIN | 50% | 90% | MAX |
| Amoxicillin | ≤0.015 | ≤0.015 | 0.03 | 0.12 |
| Augmentin | ≤0.015 | ≤0.015 | 0.03 | 0.12 |
| Ceftriaxone | ≤0.015 | 0.03 | 0.03 | 0.12 |
| Ciprofloxacin | 0.25 | 0.5 | 2 | 2 |
| Clarithromycin | ≤0.03 | ≤0.03 | 0.5 | ≥64 |
| Daptomycin | ≤0.03 | ≤0.03 | 0.06 | 0.12 |
| Ozenoxacin | 0.008 | 0.03 | 0.03 | 0.06 |
| Levofloxacin | 0.5 | 0.5 | 2 | 2 |
| Linezolid | 0.5 | 1 | 1 | 2 |
| Moxifloxacin | 0.12 | 0.25 | 0.25 | 0.5 |
| Tigecycline | 0.015 | 0.03 | 0.06 | 0.12 |

### 3.6 Activity against Group B Streptococci

Summary data for ozenoxacin and comparators against 20 recent clinical strains of Group B *Streptococci* are shown in Table 6.

From the results, ozenoxacin appears to be more active than all comparators against all strains of group B *Streptococci.* In particular, ozenoxacin is thirty-two times more active than ciprofloxacin or levofloxacin and has eight times more activity when compared to moxifloxacin. Although clarithromycin end points have not been ascertained, ozenoxacin has lower maximum MIC values.

**Table 6. Summary MIC data for ozenoxacin and comparators against 20 Group B Streptococci**

| Antimicrobial | MIC mg/L | | | |
|---|---|---|---|---|
| | MIN | 50% | 90% | MAX |
| Amoxicillin | 0.03 | 0.06 | 0,12 | 0.12 |
| Augmentin | 0.03 | 0.06 | 0,12 | 0.12 |
| Ceftriaxone | 0.03 | 0.06 | 0.12 | 0.25 |
| Ciprofloxacin | 0.5 | 0.5 | 1 | 1 |
| Clarithromycin | ≤0.03 | ≤0.03 | ≤0.03 | 4 |
| Daptomycin | 0.12 | 0.5 | 1 | 1 |
| Ozenoxacin | 0.008 | 0.008 | 0.03 | 0.03 |
| Levofloxacin | 0.5 | 0.5 | 1 | 1 |

| Linezolid | 1 | 1 | 1 | 2 |
|---|---|---|---|---|
| Moxifloxacin | 0.12 | 0.12 | 0.25 | 0.5 |
| Tigecycline | 0.03 | 0.06 | 0.06 | 0.06 |

### 3.7 Activity against Group C Streptococci

Summary data for ozenoxacin and comparators against 20 recent clinical strains of Group C *Streptococci* are shown in Table 7.

As for group B *Streptococci,* the table shows that ozenoxacin was more active than all comparators against all strains of group C *Streptococci* including clarithromycin non-susceptible strains. Most notably, ozenoxacin is seen to have thirty-two times the activity of levofloxacin, sixteen times that of ciprofloxacin and eight times that of moxifloxacin.

**Table 7. Summary MIC data for ozenoxacin and comparators against 20 Group C Streptococci**

| Antimicrobial | MIC mg/L | | | |
|---|---|---|---|---|
| | MIN | 50% | 90% | MAX |
| Amoxicillin | ≤0.015 | 0.03 | 0.03 | 0.25 |
| Augmentin | ≤0.015 | 0.03 | 0.03 | 0.03 |
| Ceftriaxorie | 0.03 | 0.06 | 0.06 | 0.12 |
| Ciprofloxacin | 0.5 | 0.5 | 0.5 | 2 |
| Clarithromycin | ≤0.03 | ≤0.03 | 8 | 32 |
| Daptomycin | ≤0.03 | 0.12 | 0.25 | 0.5 |
| Ozenoxacin | 0.008 | 0.008 | 0.03 | 0.03 |
| Levofloxacin | 0.5 | 0.5 | 1 | 1 |
| Linezolid | 0.5 | 1 | 2 | 2 |
| Moxifloxacin | 0.12 | 0.12 | 0.25 | 0.25 |
| Tigecycline | 0.03 | 0.06 | 0.25 | 0.25 |

### 3.8 Activity against Group G Streptococci

Summary data for ozenoxacin and comparators against 20 recent clinical strains of Group G *Streptococci* are shown in Table 8.

It can be seen that ozenoxacin was also highly active against all strains of group G *Streptococci* with similar activity to amoxicillin and amoxicillin-clavulanate. These data show that ozenoxacin has increased activity against all other compounds, and it has also thirty-two times increased activity compared to ciprofloxacin or levofloxacin and eight times increased activity compared to moxifloxacin.

**Table 8. Summary MIC data for ozenoxacin and comparators against 20 Group G Streptococci**

| Antimicrobial | MIC mg/l | | | |
|---|---|---|---|---|
| | MIN | 50% | 90% | MAX |
| Amoxicillin | ≤0.015 | ≤0.015 | 0.06 | 0.12 |
| Augmentin | ≤0.015 | ≤0.015 | 0.03 | 0.03 |
| Ceftnaxone | 0.03 | 0.03 | 0.06 | 0.12 |
| Ciprofloxacin | 0.25 | 0.5 | 1 | 8 |
| Clarithromycin | ≤0.03 | ≤0.03 | 2 | ≥64 |
| Daptomycin | ≤0.03 | 0.12 | 0.25 | 0.5 |
| Ozenoxacin | 0.008 | 0.008 | 0.03 | 0.25 |
| Levofloxacin | 0.25 | 0.5 | 1 | 8 |
| Linezolid | 1 | 1 | 2 | 2 |
| Moxifloxacín | 0.06 | 0.12 | 0.25 | 2 |
| Tigecycline | 0.03 | 0.06 | 0.25 | 0.25 |

### 3.9 Activity against Streptococcus constellatus

Summary data for ozenoxacin and comparators against 9 recent clinical strains of *Streptococcus constellatus* are shown in Table 9.

At the concentrations tested, the table shows that ozenoxacin is the most active compound against S. constellatus and has a very tight MIC range of 0.015-0.03mg/L. These data again show that ozenoxacin has thirty-two times increased activity compared to ciprofloxacin or levofloxacin and eight times increased activity compared to moxifloxacin.

**Table 9. Summary MIC data for ozenoxacin and comparators against 9 Streptococcus constellatus**

| Antimicrobial | MIC mg/L | | | |
|---|---|---|---|---|
| | MIN | 50% | 90% | MAX |
| Amoxicillin | 0.12 | 0.25 | 0.25 | 0.25 |
| Augmentin | 0.06 | 0.12 | 0.25 | 0.25 |
| Ceftriaxone | 0.06 | 0.25 | 0.5 | 0.5 |
| Ciprofloxacin | 0.5 | 0.5 | 1 | 1 |
| Clarithromycin | ≤0.03 | ≤0.03 | ≥64 | ≥64 |
| Daptomycin | 0.12 | 0.5 | 0.5 | 0.5 |
| Ozenoxacin | 0.015 | 0.03 | 0.03 | 0.03 |
| Levofloxacin | 0.12 | 0.5 | 1 | 1 |
| Linezolid | 0.06 | 1 | 2 | 2 |
| Moxifloxacin | 0.03 | 0.12 | 0.25 | 0.25 |
| Tigecycline | 0.015 | 0.03 | 0.06 | 0.06 |

### 3.10 Activity against Streptococcus bovis

Summary data for ozenoxacin and comparators against 10 recent clinical strains of *Streptococcus bovis* are shown in Table 10.

Ozenoxacin was found to be more active than ciprofloxacin (thirty-two times more active), levofloxacin (thirty-two times more active), moxifloxacin (four times more active), amoxicillin, augmentin, ceftriaxone, linezolid or daptomycin against S. bovis. The potency of ozenoxacin is comparable to tigecycline.

**Table 10. Summary MIC data for ozenoxacin and comparators against 10 Streptococcus bovis**

| **Antimicrobial** | **MIC mg/L** | | | |
|---|---|---|---|---|
| | **MIN** | **50%** | **90%** | **MAX** |
| Amoxicillin | ≤0.015 | 0.12 | 0.12 | 0.12 |
| Augmentin | ≤0.015 | 0.12 | 0.12 | 0.12 |
| Ceftriaxone | 0.03 | 0.12 | 0.25 | 0.25 |
| Ciprofloxacin | 0.5 | 2 | 2 | ≥64 |
| Clarithromycin | ≤0.03 | ≤0.03 | ≥64 | ≥64 |
| Daptomycin | ≤0.03 | 0.06 | 0.12 | 0.12 |
| Ozenoxacin | 0.008 | 0.03 | 0.06 | 0.5 |
| Levofloxacin | 0.25 | 1 | 2 | ≥64 |
| Linezolid | 0.5 | 1 | 1 | 1 |
| moxifloxacin | 0.06 | 0.25 | 0.25 | 8 |
| Tigecycline | 0.015 | 0.03 | 0.06 | 0.06 |

### 3.11 Activity against Streptococcus mitis

Summary data for ozenoxacin and comparators against 10 recent clinical strains of *Streptococcus mitis* are shown in Table 11.

Against isolates of S. mitis, ozenoxacin has comparable activity to tigecycline but is more active than all other comparator compounds including more than one hundred times the activity of ciprofloxacin, thirty-two times the activity of levofloxacin and four times the activity of moxifloxacin.

**Table 11. Summary MIC data for ozenoxacin and comparators against 10 Streptococcus mitis**

| **Antimicrobial** | **MIC mg/L** | | | |
|---|---|---|---|---|
| | **MIN** | **50%** | **90%** | **MAX** |
| Amoxicillin | 0.03 | 0.25 | 8 | 32 |
| Augmentin | 0.03 | 0.25 | 8 | 16 |
| Ceftriaxone | 0.03 | 0.12 | 0.5 | 1 |
| Ciprofloxacin | 0.5 | 2 | 8 | ≥64 |
| Clarithromycin | ≤0.03 | 1 | ≥64 | ≥64 |
| Daptomycin | 0.12 | 0.5 | 1 | 1 |
| Ozenoxacin | 0.015 | 0.03 | 0.06 | 0.5 |
| Levofloxacin | 0.5 | 1 | 2 | 32 |
| Linezolid | 0.5 | 1 | 1 | 1 |
| Moxifloxacin | 0.12 | 0,12 | 0,25 | 4 |
| Tigecycline | 0.015 | 0,03 | 0,12 | 0,25 |

### 3.12 Activity against Streptococcus sanguis

Summary data for ozenoxacin and comparators against 10 recent clinical strains of *Streptococcus sanguis* are shown in Table 12.

Again, ozenoxacin is more active than ciprofloxacin (over one hundred times), levofloxacin (thirty-two times), moxifloxacin (eight times), amoxicillin, augmentin, ceftriaxone, linezolid or daptomycin against S. sanguis. The potency of ozenoxacin is comparable to tigecycline.

**Table 12. Summary MIC data for ozenoxacin and comparators against 10 Streptococcus sanguis**

| **Antimicrobial** | **MIC mg/L** | | | |
|---|---|---|---|---|
| | **MIN** | **50%** | **90%** | **MAX** |
| Amoxicillin | ≤0.015 | 0.25 | 0.5 | 0,5 |
| Augmentin | ≤0.015 | 0.25 | 0.5 | 0.5 |
| Ceftriaxone | 0.03 | 0.12 | 0.25 | 0.25 |
| Ciprofloxacin | 0.5 | 1 | 4 | 8 |
| Clarithromycin | ≤0.03 | ≤0.03 | 0.5 | 2 |
| Daptomycin | 0.12 | 0.25 | 0.5 | 1 |
| Ozenoxacin | 0.015 | 0.03 | 0.03 | 0.06 |
| Levofloxacin | 0.5 | 0.5 | 1 | 2 |
| Linezolid | 0.5 | 1 | 1 | 1 |
| Moxifloxacin | 0.12 | 0.12 | 0.25 | 4 |
| Tigecycline | 0.015 | 0.03 | 0.06 | 0.06 |

### 3.13 Activity against Streptococcus oralis

Summary data for ozenoxacin and comparators against 20 recent clinical strains of *Streptococcus oralis* are shown in Table 13.

The table shows that ozenoxacin has improved activity against *S. oralis* compared to all the fluoroquinolones, ceftriaxone, linezolid and daptomycin and similar activity to amoxicillin, augmentin or tigecycline. In particular, ozenoxacin was sixty-four times more potent than ciprofloxacin, sixteen times more potent than levofloxacin and four times more potent than moxifloxacin.

**Table 13. Summary MIC data for ozenoxacin and comparators against 10 Streptococcus oralis**

| **Antimicrobial** | **MIC mg/L** | | | |
|---|---|---|---|---|
| | **MIN** | **50%** | **90%** | **MAX** |
| Amoxicillin | 0.03 | 0.06 | 0.25 | 1 |
| Augmentin | 0.03 | 0.06 | 0.5 | 1 |
| Ceftriaxone | 0.03 | 0.12 | 0.5 | 0.5 |
| Ciprofloxacin | 0.5 | 2 | 4 | 4 |
| Clarithromycin | ≤0.03 | ≤0.03 | 0.25 | 1 |
| Daptomycin | 0.25 | 0.5 | 2 | 2 |
| Ozenoxacin | 0.015 | 0.03 | 0.06 | 0.06 |
| Levofloxacin | 0.5 | 1 | 1 | 2 |
| Linezolid | 0.5 | 1 | 1 | 1 |
| Moxifloxacin | 0.06 | 0.12 | 0.25 | 1 |
| Tigecycline | 0.015 | 0.03 | 0.06 | 0.12 |

### 3.14 Activity against Streptococcus pneumoniae

Summary data for ozenoxacin and comparators against 64 recent clinical strains of *Streptococcus pneumoniae* are shown in Table 14.

This table shows that ozenoxacin is highly active against all isolates of *S. pneumoniae* and has increased activity compared to ciprofloxacin (sixty-four times increased), levofloxacin (thirty-two times increased), moxifloxacin (eight times increased), daptomycin, clarithromycin or linezolid. Compared to Beta-lactam and cephalosporin comparators, ozenoxacin has improved activity against those isolates with reduced susceptibility to penicillin. The potency of ozenoxacin is comparable to tigecycline.

**Table 14. Summary MIC data for ozenoxacin and comparators against 64 Streptococcus pneumoniae**

| **Antimicrobial** | **MIC mg/L** | | | |
|---|---|---|---|---|
| | **MIN** | **50%** | **90%** | **MAX** |
| Amoxicillin | ≤0.015 | 0.25 | 8 | 16 |
| Augmentin | ≤0.015 | 0.12 | 8 | 8 |
| Ceftriaxone | ≤0.015 | 0.25 | 2 | 4 |
| Ciprofloxacin | 0.5 | 1 | 2 | 2 |
| Clarithromycin | ≤0,03 | 0.06 | ≥64 | ≥64 |
| Daptomycin | ≤0.03 | 0.12 | 0.5 | 0.5 |
| Ozenoxacin | 0.015 | 0.03 | 0.03 | 0.06 |
| Levofloxacin | 0.5 | 1 | 1 | 1 |
| Linezolid | 0.25 | 1 | 1 | 2 |
| Moxifloxacin | 0.06 | 0.12 | 0.25 | 0.25 |
| Tigecycline | 0.015 | 0.03 | 0.03 | 0.06 |

### 3.15 Activity against Listeria monocytogenes

Summary data for ozenoxacin and comparators against 20 library strains of *Listeria monocytogenes* are shown in Table 15.

When tested against isolates of *L. monocytogenes,* ozenoxacin was the most active compound of all those tested and has an MIC₉₀ of 0.06mg/L. These data show that ozenoxacin was sixteen times more active than ciprofloxacin and eight times more active than moxifloxacin against these strains.

**Table 15. Summary MIC data for ozenoxacin and comparators against 20 Listeria monocytogenes**

| **Antimicrobial** | **MIC mg/L** | | | |
|---|---|---|---|---|
| | **MIN** | **50%** | **90%** | **MAX** |
| Amoxicillin | 0.06 | 0.25 | 0.5 | 0.5 |
| Augmentin | 0.06 | 0.25 | 0.5 | 0.5 |
| Ceftriaxone | 1 | 2 | ≥64 | ≥64 |
| Ciprofloxacin | 0.5 | 0.5 | 1 | 2 |
| Clarithromycin | ≤0.03 | 0,06 | 0,12 | 4 |
| Daptomycin | 0.015 | 1 | 2 | ≥128 |
| Ozenoxacin | 0.008 | 0.03 | 0.06 | 0.12 |
| Levofloxacin | 0.25 | 1 | 1 | 2 |
| Linezolid | 1 | 2 | 2 | 8 |
| Moxifloxacin | 0.12 | 0.25 | 0.5 | 1 |
| Tigecycline | 0.015 | 0.06 | 0.12 | 0.12 |

### 3.16 Activity against Escherichia coli

Summary data for ozenoxacin and comparators against 50 recent clinical strains of *Escherichia coli* are shown in Table 16.

Ozenoxacin was less active than ciprofloxacin, levofloxacin or moxifloxacin against strains of *E. coli.* Nonetheless, it was still found to be more active than amoxicillin, augmentin, linezolid, daptomycin and clarithromycin comparators. Ceftriaxone and tigecycline were the most active compounds against tested *E. coli* strains.

**Table 16. Summary MIC data for ozenoxacin and comparators against 50 Escherichia coli**

| **Antimicrobial** | **MIC mg/L** | | | |
|---|---|---|---|---|
| | **MIN** | **50%** | **90%** | **MAX** |
| Amoxicillin | 4 | 16 | ≥64 | ≥64 |
| Augmentin | 2 | 8 | 16 | ≥64 |
| Ceftriaxone | ≤0.015 | 0.06 | 0.12 | ≥64 |
| Ciprofloxacin | ≤0.015 | ≤0.015 | 32 | ≥64 |
| Clarithromycin | 16 | ≥64 | ≥64 | ≥64 |
| Daptomycin | ≥128 | ≥128 | ≥128 | ≥128 |
| Ozenoxacin | 0.008 | 0.06 | ≥128 | ≥128 |
| Levofloxacin | ≤0.015 | 0.03 | 16 | ≥64 |
| Linezolid | 16 | ≥128 | ≥128 | ≥128 |
| moxifloxacin | 0.03 | 0.06 | 32 | ≥64 |
| Tigecycline | 0.06 | 0.12 | 0.25 | 0.5 |

### 3.17 Activity against Pseudomonas aeruginosa

Summary data for ozenoxacin and comparators against 50 recent clinical strains of *Pseudomonas aeruginosa* are shown in Tables 17.

Against strains of *P. aeruginosa,* ozenoxacin has similar activity to moxifloxacin but weaker activity than levofloxacin or ciprofloxacin. Ozenoxacin has improved activity compared to amoxicillin, augmentin, ceftriaxone, tigecycline, linezolid, clarithromycin or daptomycin.

**Table 17. Summary MIC data for ozenoxacin and comparators against 50 Pseudomonas aeruqinosa**

| **Antimicrobial** | **MIC mg/L** | | | |
|---|---|---|---|---|
| | **MIN** | **50%** | **90%** | **MAX** |
| Amoxicillin | ≥64 | ≥64 | ≥64 | ≥64 |
| Augmentin | ≥64 | ≥64 | ≥64 | ≥64 |
| Ceftriaxone | 8 | ≥64 | ≥64 | ≥64 |
| Ciprofloxacin | 0.06 | 0.25 | 16 | ≥64 |
| Clarithromycin | ≥64 | ≥64 | ≥64 | ≥64 |
| Daptomycin | ≥128 | ≥128 | ≥128 | ≥128 |
| Ozenoxacin | 0.5 | 4 | ≥128 | ≥128 |
| Levofloxacin | 0.25 | 1 | 32 | ≥64 |
| Linezolid | ≥128 | ≥128 | ≥128 | ≥128 |
| Moxifloxacin | 1 | 2 | ≥64 | ≥64 |
| Tigecycline | 0.5 | 8 | ≥16 | ≥16 |

### 3.18 Activity against Salmonella spp.

Summary data for ozenoxacin and comparators against 20 library strains of *Salmonella spp.* are shown in Table 18.

Isolates of *Salmonella spp.* are more susceptible to ozenoxacin than amoxicillin, augmentin, tigecycline, linezolid, daptomycin or clarithromycin. Ozenoxacin showed similar potency to these strains as levofloxacin, moxifloxacin or ceftriaxone and weaker activity than ciprofloxacin.

**Table 18. Summary MIC data for ozenoxacin and comparators against 20 Salmonella spp.**

| **Antimicrobial** | **MIC mg/L** | | | |
|---|---|---|---|---|
| | **MIN** | **50%** | **90%** | **MAX** |
| Amoxicillin | 1 | 1 | ≥64 | ≥64 |
| Augmentin | 1 | 1 | 8 | 32 |
| Ceftriaxone | ≤0.015 | 0.12 | 0.12 | 1 |
| Ciprofloxacin | ≤0.015 | ≤0.015 | ≤0.015 | ≤0.015 |
| Clarithromycin | 16 | ≥64 | ≥64 | ≥64 |
| Daptomycin | ≥128 | ≥128 | ≥128 | ≥128 |
| Ozenoxacin | 0.03 | 0.12 | 0.12 | 0.25 |
| Levofloxacin | ≤0.015 | 0.06 | 0.06 | 0.12 |
| Linezolid | 4 | ≥128 | ≥128 | ≥128 |
| Moxifloxacin | ≤0.015 | 0.06 | 0.12 | 0.12 |
| Tigecycline | 0.12 | 0.5 | 2 | 4 |

### 3.19 Activity against Shigella spp.

Summary data for ozenoxacin and comparators against 20 library strains of *Shigella spp.* are shown in Table 19.

This table shows that there is comparable activity between ozenoxacin and moxifloxacin against isolates of *Shigella spp.* but weaker activity than ciprofloxacin or levofloxacin. Ozenoxacin also shows improved potency compared to amoxicillin, augmentin, ceftriaxone, tigecycline, linezolid, daptomycin or clarithromycin.

**Table 19. Summary MIC data for ozenoxacin and comparators against 20 Shigella spp.**

| **Antimicrobial** | **MIC mg/L** | | | |
|---|---|---|---|---|
| | **MIN** | **50%** | **90%** | **MAX** |
| Amoxicillin | 1 | 8 | ≥64 | ≥64 |
| Augmentin | 1 | 8 | 16 | 32 |
| Ceftriaxone | 0.03 | 0.06 | 0.06 | 1 |
| Ciprofloxacin | ≤0.015 | ≤0.015 | ≤0.015 | ≤0.015 |
| Clarithromycin | 16 | 32 | ≥64 | ≥64 |
| Daptomycin | ≥128 | ≥128 | ≥128 | ≥128 |
| Ozenoxacin | 0.008 | 0.03 | 0.03 | 0.03 |
| Levofloxacin | ≤0.015 | ≤0.015 | ≤0.015 | ≤0.015 |
| Linezolid | 64 | ≥128 | ≥128 | ≥128 |
| Moxifloxacin | ≤0.015 | 0.03 | 0.03 | 0.03 |
| Tigecycline | 0.12 | 0.5 | 2 | 2 |

### 3.20 Activity against Haemophilus influenzae

Summary data for ozenoxacin and comparators against 69 recent clinical strains of *Haemophilus influenzae* are shown in Table 20.

These data show that ozenoxacin has similar activity against Beta-lactamase-positive, -negative and non-typeable strains of *H. influenzae* to all fluoroquinolones or ceftriaxone. Ozenoxacin MICs were as low as 0.002mg/L, with an MIC₅₀ of just 0.004mg/L. The compound was more active than all other comparators.

**Table 20. Summary MIC data for ozenoxacin and comparators against 70 Haemophilus influenzae**

| **Antimicrobial** | **MIC mg/L** | | | |
|---|---|---|---|---|
| | **MIN** | **50%** | **90%** | **MAX** |
| Amoxicillin | 0.12 | 1 | ≥64 | ≥64 |
| Augmentin | 0.25 | 0.5 | 1 | 4 |
| Ceftriaxone | ≤0.015 | ≤0.015 | ≤0.015 | 0.25 |
| Ciprofloxacin | ≤0.015 | ≤0.015 | ≤0.015 | 0,5 |
| Clarithromycin | 2 | 8 | 8 | 16 |
| Daptomycin | ≥128 | ≥128 | ≥128 | ≥128 |
| Ozenoxacin | 0.002 | 0.004 | 0.015 | 0.25 |
| Levofloxacin | ≤0.015 | ≤0.015 | ≤0.015 | 0.25 |
| Linezolid | 8 | 16 | 16 | 32 |
| Moxifloxacin | ≤0.015 | ≤0.015 | 0.06 | 1 |
| Tigecycline | 0.12 | 0.25 | 0,5 | 1 |

### 3.21 Activity against Moraxella catarrhalis

Summary data for ozenoxacin and comparators against 50 recent clinical strains of *Moraxella catarrhalis* are shown in Table 21.

The table shows that ozenoxacin is highly potent against both Beta-lactamase-positive and -negative strains of *M. catarrhalis,* with MIC lower than 0.0005mg/L. These data show that ozenoxacin has eight times improved activity compared to moxifloxacin and levofloxacin, and four times improved activity compared to ciprofloxacin. It was also more active than all other comparators.

**Table 21. Summary MIC data for ozenoxacin and comparators against 50 Moraxella catarrhalis**

| Antimicrobial | MIC mg/L | | | |
|---|---|---|---|---|
| | MIN | 50% | 90% | MAX |
| Amoxicillin | ≤0.015 | 0.12 | 8 | 16 |
| Augmentin | ≤0.015 | 0.03 | 0.25 | 0.25 |
| Ceftriaxone | ≤0.015 | 0.06 | 1 | 2 |
| Ciprofloxacin | ≤0.015 | 0.03 | 0.03 | 0.06 |
| Clarithromycin | ≤0.03 | 0.06 | 0.06 | 0.12 |
| Daptomycin | 2 | 8 | 16 | ≥128 |
| Ozenoxacin | ≤0.0005 | 0.004 | 0.008 | 0.015 |
| Levofloxacin | ≤0.015 | 0.06 | 0.06 | 0.06 |
| Linezolid | 4 | 4 | 4 | 8 |
| Moxifloxacin | 0.03 | 0.06 | 0.06 | 0.12 |
| Tigecycline | 0.03 | 0.06 | 0.06 | 0.25 |

### 3.22 Activity against Neisseria gonorrhoeae

Summary data for ozenoxacin and comparators against 20 library strains of *Neisseria gonorrhoeae* are shown in Table 22.

These data again show ozenoxacin to be the most highly active compound, producing MIC as low as ≤0.0005mg/L, along with an MIC₅₀ and MIC₉₀ of 0.004 and 0.008mg/L respectively. Ozenoxacin is shown to be eight times more potent than moxifloxacin, and although ciprofloxacin and levofloxacin end points have not been ascertained, ozenoxacin has lower maximum MIC values.

**Table 22. Summary MIC data for ozenoxacin and comparators against 20 Neisseria gonorrhoeae**

| **Antimicrobial** | **MIC mg/L** | | | |
|---|---|---|---|---|
| | **MIN** | **50%** | **90%** | **MAX** |
| Amoxicillin | ≤0.03 | 0.5 | 16 | ≥64 |
| Augmentin | 0.06 | 0.5 | 1 | 2 |
| Ceftriaxone | ≤0.03 | ≤0.03 | ≤0.03 | ≤0.03 |
| Ciprofloxacin | ≤0.03 | ≤0.03 | ≤0.03 | 16 |
| Clarithromycin | ≤0.03 | 0.5 | 2 | 8 |
| Daptomycin | 64 | ≥128 | ≥128 | ≥128 |
| Ozenoxacin | ≤0.0005 | 0.004 | 0.008 | 0.12 |
| Levofloxacin | ≤0.03 | ≤0.03 | ≤0.03 | 16 |
| Linezolid | 0.25 | 1 | 1 | 8 |
| Moxifloxacin | ≤0.03 | ≤0.03 | 0.06 | 4 |
| Tigecycline | ≤0.03 | 0.25 | 0.5 | 1 |

### 3.23 Activity against Neisseria meningitidis

Summary data for ozenoxacin and comparators against 20 library strains of *Neisseria meningitidis* are shown in Table 23.

Against strains of *N. meningitidis* ozenoxacin was again the most highly potent with many MICs lower than 0.0005mg/L. Ozenoxacin is significantly more active than all other fluoroquinolones, amoxicillin, augmentin, tigecycline, linezolid or daptomycin, with sixteen times the activity of the other fluoroquinolones.

**Table 23. Summary MIC data for ozenoxacin and comparators against 20 Neisseria meninqitidis**

| **Antimicrobial** | **MIC mg/L** | | | |
|---|---|---|---|---|
| | **MIN** | **50%** | **90%** | **MAX** |
| Amoxicillin | ≤0.015 | 0.03 | 0.12 | 0.25 |
| Augmentin | ≤0.015 | 0.03 | 0.12 | 0.25 |
| Ceftriaxone | ≤0.015 | ≤0.015 | ≤0.015 | 0.06 |
| Ciprofloxacin | ≤0.015 | ≤0.015 | 0.03 | 0.06 |
| Clarithromycin | ≤0.03 | ≤0.03 | 0.25 | 1 |
| Daptomycin | 8 | 64 | ≥128 | ≥128 |
| Ozenoxacin | ≤0.0005 | 0.002 | 0.002 | 0.008 |
| Levofloxacin | ≤0.015 | ≤0.015 | 0.03 | 0.03 |
| Linezolid | 4 | 8 | 8 | 8 |
| Moxifloxacin | ≤0.015 | ≤0.015 | 0.03 | 0.06 |
| Tigecycline | 0.008 | 0.06 | 0.12 | 0.25 |

### 3.24 Activity against Helicobacter pylori

Summary data for ozenoxacin and comparators against 5 library strains of *Helicobacter pylori* are shown in Tables 24.

Ozenoxacin is also very active against all *H. pylori* isolates tested and with considerably more activity than the other fluoroquinolones at over one hundred times the activity of ciprofloxacin and levofloxacin and sixty-four times that of moxifloxacin. The maximum ozenoxacin MIC was only 0.008mg/L, and it was more active than all other compounds.

**Table 24. Summary MIC data for ozenoxacin and comparators against 5 Helicobacter pylori**

| **Antimicrobial** | **MIC mg/L** | | | |
|---|---|---|---|---|
| | **MIN** | **50%** | **90%** | **MAX** |
| Amoxicillin | ≤0.03 | ≤0.03 | ≤0.03 | ≤0.03 |
| Augmentin | ≤0.03 | ≤0.03 | ≤0.03 | ≤0.03 |
| Ceftriaxone | 0.12 | 0.25 | 0.25 | 0.25 |
| Ciprofloxacin | 0.12 | 0.5 | 1 | 1 |
| Clarithromycin | ≤0.03 | ≤0.03 | 32 | 32 |
| Daptomycin | ≥128 | ≥128 | ≥128 | ≥128 |
| Ozenoxacin | 0.002 | 0.008 | 0.008 | 0.008 |
| Levofloxacin | 0.25 | 0.25 | 1 | 1 |
| Linezolid | 8 | 8 | 8 | 8 |
| Moxifloxacin | 0.12 | 0.5 | 0.5 | 0.5 |
| Tigecycline | ≤0.03 | 0.12 | 0.12 | 0.12 |

### 3.25 Activity against Bacteroides fragilis

Summary data for ozenoxacin and comparators against 20 library strains of *Bacteroides fragilis* are shown in Table 25.

The table shows that ozenoxacin had significantly more activity than any other compound tested against *B. fragilis* including over one hundred times more activity than ciprofloxacin, thirty-two times more activity than levofloxacin and sixteen times more activity than moxifloxacin.

**Table 25. Summary MIC data for ozenoxacin and comparators against 20 Bacteroides fragilis**

| **Antimicrobial** | **MIC mg/L** | | | |
|---|---|---|---|---|
| | **MIN** | **50%** | **90%** | **MAX** |
| Amoxicillin | 2 | 32 | ≥64 | ≥64 |
| Augmentin | 0.12 | 0.5 | 16 | 16 |
| Ceftriaxone | 0.5 | ≥64 | ≥64 | ≥64 |
| Ciprofloxacin | 2 | 4 | 16 | 32 |
| Clarithromycin | 0.25 | 1 | 2 | 8 |
| Daptomycin | ≥128 | ≥128 | ≥128 | ≥128 |
| Ozenoxacin | 0.015 | 0.12 | 0.12 | 1 |
| Levofloxacin | 0.25 | 1 | 4 | 32 |
| Linezolid | 2 | 4 | 4 | 4 |
| Moxifloxacin | 0.12 | 0.5 | 2 | 8 |
| Tigecydine | 0.25 | 0.5 | 1 | 8 |

### 3.26 Activity against Clostridium difficile

Summary data for ozenoxacin and comparators against 10 library strains of *Clostridium difficile* are shown in Table 26.

Ozenoxacin shows comparable activity to C. *ditficile* isolates as amoxicillin, augmentin and linezolid. It also shows vastly improved activity compared to other fluoroquinolones and ceftriaxone, with at least thirty-two times the activity of levofloxacin and ciprofloxacin, and sixteen times the activity of moxifloxacin against these isolates. Tigecycline is the most active compound against tested *C. ditficile* strains.

**Table 26. Summary MIC data for ozenoxacin and comparators against 10 Clostridium difficile**

| **Antimicrobial** | **MIC mg/L** | | | |
|---|---|---|---|---|
| | **MIN** | **50%** | **90%** | **MAX** |
| Amoxicillin | 0.5 | 1 | 2 | 2 |
| Augmentin | 1 | 2 | 2 | 2 |
| Ceftriaxone | 32 | ≥64 | ≥64 | ≥64 |
| Ciprofloxacin | 16 | ≥64 | ≥64 | ≥64 |
| Clarithromycin | 1 | ≥64 | ≥64 | ≥64 |
| Daptomycin | 0.12 | 0.25 | 2 | 2 |
| Ozenoxacin | 0.25 | 2 | 2 | 2 |
| Levofloxacin | 4 | ≥64 | ≥64 | ≥64 |
| Linezolid | 2 | 2 | 2 | 2 |
| Moxifloxacin | 2 | 32 | 32 | 32 |
| Tigecycline | ≤0.03 | ≤0.03 | 0.06 | 0.06 |

### 3.27 Activity against Clostridium perfringens

Summary data for ozenoxacin and comparators against 10 library strains of *Clostridium perfringens* are shown in Table 27.

Against strains of *C. perfringens,* ozenoxacin was again highly potent with MIC₉₀ at 0.015mg/L. Ozenoxacin is notably more active than all comparator compounds against the tested strains, showing thirty-two times the activity of moxifloxacin and ciprofloxacin, and sixteen times the activity of levofloxacin.

**Table 27. Summary MIC data for ozenoxacin and comparators against 10 Clostridium periringens**

| **Antimicrobial** | **MIC mg/L** | | | |
|---|---|---|---|---|
| | **MIN** | **50%** | **90%** | **MAX** |
| Amoxicillin | ≤0.03 | ≤0.03 | 0.06 | 0.12 |
| Augmentin | ≤0.03 | ≤0.03 | 0.12 | 0.12 |
| Ceftriaxone | ≤0.03 | 0.12 | 2 | 2 |
| Ciprofloxacin | 0.25 | 0.5 | 0.5 | 0.5 |
| Clarithromycin | 0.25 | 0.5 | 1 | 1 |
| Daptomycin | 0.12 | 1 | 2 | 2 |
| Ozenoxacin | 0.015 | 0.015 | 0.015 | 0.12 |
| Levofloxacin | 0.25 | 0.25 | 0.25 | 0.5 |
| Linezolid | 1 | 2 | 2 | 4 |
| Moxifloxacin | 0.25 | 0.5 | 0.5 | 0.5 |
| Tigecycline | 0.06 | 0.25 | 0.5 | 0.5 |

### 4. Conclusions

Ozenoxacin was highly active against all groups of pathogens tested and was the most active compound against strains of Group B *Streptococci,* Group C *Streptococci, L. monocytogenes, M. catarrhalis, B. fragilis* and C. *perfringens.* Aside from these isolates, ozenoxacin had improved activity compared to all fluoroquinolones comparators amongst all strains of MRSA, MSSA, MRSE, MSSE, Group A *Streptococci,* Group G *Streptococci, S. constellatus, S. bovis, S. mitis, S. oralis, S. pneumoniae, N. meningitidis, H. pylori and C. difficile.*

### Example 2: In vitro microbiological activity against atypical bacteria

### 1. Purpose

The aim of the study was to evaluate the MIC for ozenoxacin comparatively to reference antimicrobial agents against atypical pathogens such as *Chlamydia trachomatis, Chlamydophila pneumoniae, Mycoplasma pneumoniae, Mycoplasma hominis, Ureaplasma urealyticum, Mycobacterium tuberculosis* and *Legionella pneumophila.*

### 2. Materials and methods

### 2.1 Antimicrobials

Reference antimicrobial compounds were selected from rifampicin and levofloxacin.

The antibacterial containing solutions were prepared as follows.

### 2.1.1 Ozenoxacin,

An ozenoxacin stock solution was prepared as described in Example 1.

### 2.1.2 Levofloxacin

A levofloxacin stock solution were prepared as described in Example 1.

### 2.1.3 Rifampicin

A rifampicin stock solution of 640 mg/L was prepared in methanol and then further diluted in sterile distilled water and test medium, as required.

### 2.2 Bacterial isolates

The following bacterial isolates were tested:
1. 14 clinical strains of *Chlamydia trachomatis* (all from the UK, 10 isolated in 2003/4, 1 isolated in 2001, 1 isolated in 1999 and 2 isolated pre-1997)
2. 5 isolates of *Chlamydophila pneumoniae* (G.R. Micro Ltd. library strains)
3. 20 isolates of *Mycoplasma pneumoniae* (G.R. Micro Ltd. library strains)
4. 27 isolates of *Mycoplasma hominis* (G.R. Micro Ltd. library strains)
5. 20 isolates of *Ureaplasma urealyticum* (G.R. Micro Ltd. library strains)
6. 10 isolates of *Mycobacterium tuberculosis* (G.R. Micro Ltd. library strains)
7. 26 isolates of *Legionella pneumophila* (15 isolated in 1999 (10 UK, 3 Sweden & 2 Hungary), 10 isolates from Spain in 2005 and the NCTC 11192 reference strain).

### 2.3 MIC determinations

a) *C. pneumoniae* or *C. trachomatis*
   - MIC was determined in 96 well flat bottom micro-titre tissue culture plates using a McCoy cell culture line in a supplemented minimal growth medium comprising of Eagle's minimum essential medium (MEM) (Invitrogen Ltd., Paisley, UK). Inclusions were demonstrated by immunofluorescence staining of cell layers on the flat base of each well. MIC was taken as the lowest concentration of antimicrobial completely inhibiting the formation of normal inclusions.
b) *Mycoplasma spp.* and *U. urealyticum*
   - MIC determinations were carried out using a broth microdilution method in Mycoplasma Broth Base (Oxoid Ltd, Hampshire, UK), prepared according to the manufacturer's instructions.
c) *M. tuberculosis*
   - MIC determinations were carried out using Clinical and Laboratory Standards Institute (CLSI, formerly NCCLS) agar dilution methodology (NCCLS (2003) Susceptibility Testing of Mycobacteria, Nocardiae and other aerobic Actinomycetes; Approved Standard. NCCLS Document M24-A. NCCLS, Wayne, Pennyslvania 19087-1898, USA.)
d) *L. pneumophila*
   - MIC determinations were carried out using an agar incorporation technique in bacteriological agar supplemented with yeast extract, Legionella BCYE growth supplement and water lysed horse blood.

### 3. Results

### 3.1 Activity against Chlamydophila pneumoniae and Chlamydia trachomatis

Ozenoxacin was more active than levofloxacin against all isolates. The level of activity was over 30-times higher than levofloxacin against the same strains.

### 3.2 Activity against Mycoplasma spp. and Ureaplasma urealyticum

Both *Mycoplasma spp.* were more susceptible to ozenoxacin than levofloxacin, with *M. hominis* being more susceptible to ozenoxacin than M. pneumoniae. Ozenoxacin was more potent against *U. urealyticum* than levofloxacin.

### 3.3 Activity against Mycobacterium tuberculosis

The activity of ozenoxacin against *M. tuberculosis* was comparable to that of rifampicin.

### 3.4 Activity against Legionella pneumophila

Both quinolones were highly active against *L. pneumophila* but again, ozenoxacin, was the most active.

### 4. Conclusions

Ozenoxacin was highly active against all the atypical pathogens tested, with better in vitro activity than levofloxacin (and similar activity to rifampicin against M. *tuberculosis*).

Summary data for ozenoxacin and comparators against all atypical pathogens are shown in Table 28.

**Table 28: Summary MIC data for ozenoxacin and levofloxacin or rifampicin against atypical pathogens**

| Pathogen (N) | Antimicrobial | Antimicrobial MIC (mg/L) | | |
|---|---|---|---|---|
| | | 50% | 90% | Range |
| *C. trachomatis* (14) | Ozenoxacin | - | ≤0.06 | ≤0.06 |
| | Levofloxacin | - | 0.12 | 0.12 - 0.25 |
| *C. pneumoniae* (5) | Ozenoxacin | - | 0.015 | 0.015 |
| | Levofloxacin | - | 0.5 | 0.5 |
| *M. pneumoniae* (20) | Ozenoxacin | 0.06 | 0.06 | 0.06 - 0.25 |
| | Levofloxacin | 0.5 | 1 | 0.25 - 4 |
| *M. hominis* (27) | Ozenoxacin | 0.03 | 0.12 | 0.03 - 0.12 |
| | Levofloxacin | 0.25 | 0.5 | 0.06 - 2 |
| *U. ureaplasma* (20) | Ozenoxacin | 0.06 | 0.12 | 0.015 - 0.25 |
| | Levofloxacin | 0.5 | 1 | 0.5 - 2 |
| M. *tuberculosis* (12) | Ozenoxacin | - | 0.25 | 0.12 - 0.25 |
| | Rifampicin | - | 0.5 | 0.06 - 2 |
| *L. pneumophila* (26) | Ozenoxacin | 0.001 | 0.001 | ≤0.0005 - 0.002 |
| | Levofloxacin | 0.008 | 0.008 | 0.004 - 0.008 |

### Example 3: Preparation of ozenoxacin tablets (400mg, 200mg, and 100mg)

a) Composition

**Table 29**

| Composition per tablet | Ozenoxacin 400mg | Ozenoxacin 200mg | Ozenoxacin 100mg |
|---|---|---|---|
| | (%) | (%) | (%) |
| Active Pharmaceutical ingredient | | | |
| Ozenoxacin | 400 mg | 200 mg | 100 mg |
| | (57.14%) | (57.14%) | (57.14%) |
| Carrier | | | |
| Mannitol (Pearlitol®160C) | 100 mg | 50 mg | 25 mg |
| | (14.29%) | (14.29%) | (14.29%) |
| Pregelatinized starch (Starch 1500®) | 35 mg | 17.5 mg | 8.75 mg |
| | (5%) | (5%) | (5%) |
| Low-substituted Hydroxypropyl cellulose (L-HPC-LH-11®) | 30 mg | 15 mg | 7.5 mg |
| | (4.29%) | (4.29%) | (4.29%) |
| Sodium stearyl fumarate (Pruv®) Sodium stearyl fumarate(Pruv®) | 10 mg | 5 mg | 2.5 mg |
| | (1.43%) | (1.43%) | (1.43%) |
| Calcium hydrogen phosphate dihydrate (Emcompress®) | 125 mg | 62.5 mg | 31.25 mg |
| | (17.85%) | (17.85%) | (17.85%) |
| Purified water | | | |
| | 200 mg | 100 mg | 50 mg |
| TOTAL TABLET WEIGHT | 700 mg | 350 mg | 175 mg |

b) Manufacturing
1. Ozenoxacin, mannitol, pregelatinized starch, and low-substituted hydroxypropyl cellulose were weighted.
2. Ozenoxacin, mannitol, pregelatinized starch and low-substituted hydroxypropyl cellulose were passed through a 0.5 mm mesh hand screen.
3. The mixture was pre-blended for 3 minutes with the high shear mixer, with impeller setting at 200-500 rpm and chopper setting at 1500 rpm.
4. Purified water was added for 2 minutes with the same impeller and chopper conditions.
5. The wet granulate was passed through the granulator with a 2 mm mesh.
6. The product was dried in a fluid bed dryer at 50°C for 1 hour. The loss on drying of granulate was checked and recorded its percentage (spec ≤ 4%).
7. The dried product was passed through a 0.6 mm mesh hand screen. Its weight was recorded and the yield calculated.
8. According to the mass reconciliation, final weights were recalculated for the extragranular excipients (sodium stearyl fumarate and calcium hydrogen phosphate dihydrate).
9. Sodium stearyl fumarate and calcium hydrogen phosphate dihydrate were weighted and the weights recorded.
10.Sodium stearyl fumarate and calcium hydrogen phosphate dihydrate were passed through a 0.5 mm mesh hand screen.
11. The pre-blend of step 9 was added to the blender together with the final dried granule obtained in step 7. The mixture was blended for 5 minutes at high speed.
12. The final powder of step 11 was tabletted in a tablet press, with punches with capsule form at a compression force as follows:
   a. 400 mg strength: capsular punches of 18x8 mm. Tablet of 700 mg weight, hardness > 150 N.
   b. 200 mg strength: capsular punches of 12.5 x 7 mm. Tablet of 350 mg weight, hardness > 120 N
   c. 100 mg strength: capsular punches of 10x6 mm. Tablet of 175 mg weight, hardness >75 N.
13. Pharmacotechnical characteristics of the tablets were recorded.
14. Final tablets were packed into aluminium/aluminium blisters.

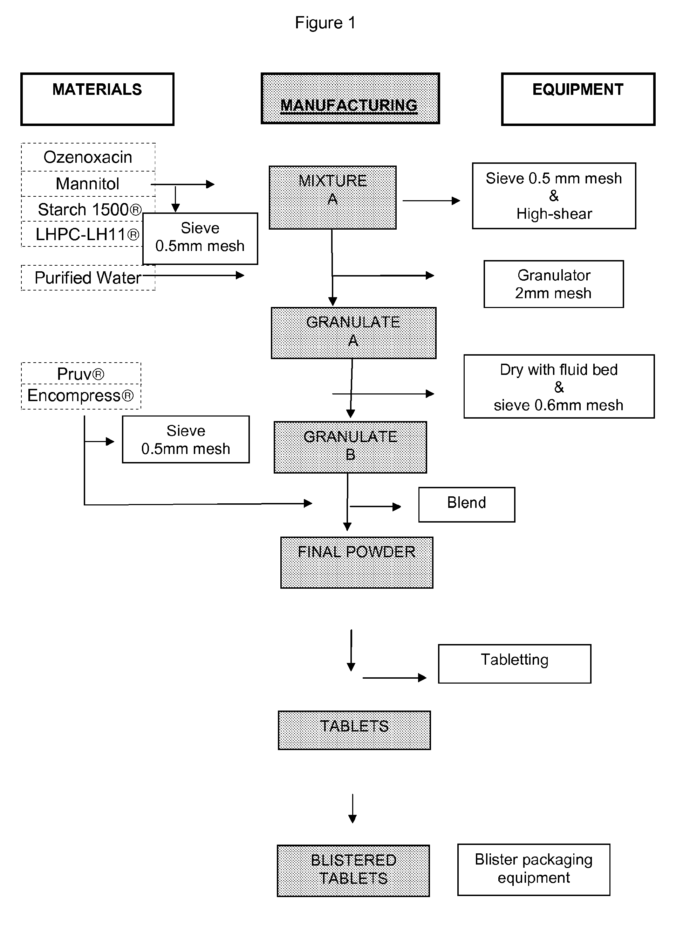

The manufacturing process for the ozenoxacin tablets including each of the steps of the manufacturing process and the equipment used in every step is shown in Figure 1.

### Example 4: Pharmacokinetic data

A pharmacokinetic study in beagle dogs showed that administration of a 400mg ozenoxacin tablet of Example 3 provides effective therapeutic levels of active ingredient during at least one 24 hour period of time against pathogenic bacteria such as non-typeable strains of *Haemophilus influenzae,* Beta-lactamase positive *Moraxella catarrhalis,* methicillin-susceptible *Staphylococcus aureus* (MSSA), *Staphylococcus epidermidis, Streptococcus pyogenes,* and *Streptococcus pneumoniae.*

## Claims

1. Use of ozenoxacin in the manufacture of a solid oral dosage form for treating or preventing systemic bacterial infections in a mammal caused by pathogenic bacteria susceptible to ozenoxacin, wherein said solid oral dosage form comprises an amount of 25 to 1200mg of active ingredient, and a suitable carrier.

2. Use according to claim 1 wherein the amount of active ingredient is 50 to 800mg.

3. Use according to claim 1 or 2 wherein the suitable carrier comprises a mixture of:
(i) a water soluble inert filler selected from mannitol, xylitol, sucrose, lactose compounds, maltodextrin, dextran, dextrin, modified starches, dextrose, sorbitol, and dextrates, and mixtures thereof, in a proportion of 5 to 70% in relation to the total weight of the dosage form;
(ii) a disintegrant selected from pregelatinized starch, low-substituted hydroxypropyl cellulose, cross-linked sodium salt of a carboxymethyl ether of starch, cross-linked sodium carboxymethyl cellulose, cross-linked polyvinylpyrrolidone, sodium starch glycolate, sorbitan fatty acid esters, docusate sodium, polyoxyethylene sorbitan fatty acid esters, and microcrystalline cellulose, and mixtures thereof, in a proportion of 2 to 30% in relation to the total weight of the dosage form;
(iii) a lubricant selected from magnesium stearate, calcium stearate, glyceryl monostearate, glyceryl palmitostearate, hydrogenated castor oil, colloidal silicon dioxide, glycerin, hydrogenated vegetable oil, mineral oil, polyethylene glycol, sodium benzoate, sodium lauryl sulfate, sodium stearyl fumarate, stearic acid, talc, and zinc stearate, and mixtures thereof, in a proportion of 0.2 to 10% in relation to the total weight of the dosage form; and
(iv) a direct compression excipient selected from calcium hydrogen phosphate hydrates, spray-dried maltose, starch acetates, poly-lactic acid, and microcrystalline cellulose, and mixtures thereof, in a proportion of 5 to 70% in relation to the total weight of the dosage form.

4. Use according to claim 3 wherein
(i) the water soluble inert filler is mannitol in a proportion of 8 to 30% in relation to the total weight of the dosage form;
(ii) the disintegrant is a mixture of pregelatinized starch in a proportion of 3 to 10% in relation to the total weight of the dosage form, and low-substituted hydroxypropyl cellulose in a proportion of 3 to 10% in relation to the total weight of the dosage form;
(iii) the lubricant is sodium stearyl fumarate in a proportion of 0.5 to 5% in relation to the total weight of the dosage form;
(iv) and the direct compression excipient is calcium hydrogen phosphate dihydrate in a proportion of 8 to 30% in relation to the total weight of the dosage form.

5. Use according to claim 4 wherein
(i) the proportion of mannitol is from 10 to 20% in relation to the total weight of the dosage form;
(iia) the proportion of pregelatinized starch is from 3 to 7% in relation to the total weight of the dosage form;
(iib) the proportion of low-substituted hydroxypropyl cellulose is from 3 to 7% in relation to the total weight of the dosage form;
(iii) the proportion of sodium stearyl fumarate is from 1 to 3% in relation to the total weight of the dosage form; and
(iv) the proportion of calcium hydrogen phosphate dihydrate is from 10 to 25% in relation to the total weight of the dosage form.

6. Use according to any one of claims 1 to 5 wherein the systemic bacterial infections are skin and skin structure's infections caused by methicillin-susceptible *Staphylococcus aureus* (MSSA), methicillin-resistant *Staphylococcus aureus* (MRSA) including ciprofloxacin-resistant strains, methicillin-susceptible *Staphylococcus epidermidis* (MSSE), methicillin-resistant *Staphylococcus epidermidis* (MRSE), *Streptococcus pyogenes,* and Group G *Streptococci.*

7. Use according to any one of claims 1 to 5 wherein the systemic bacterial infections are respiratory tract infections caused by methicillin-susceptible *Staphylococcus aureus* (MSSA), methicillin-resistant *Staphylococcus aureus* (MRSA) including ciprofloxacin-resistant strains, penicillin-resistant *Streptococcus pneumoniae,* Beta-lactamase positive *Haemophilus influenzae,* non-typeable strains of *Haemophilus influenzae,* Beta-lactamase positive *Moraxella catarrhalis, Neisseria meningitidis, Legionella pneumophila, Mycoplasma pneumoniae, Legionella pneumophila,* and *Mycobacterium tuberculosis.*

8. Use according to any one of claims 1 to 5 wherein the systemic bacterial infections are sexually transmitted diseases and genital tract infections caused by *Streptococcus agalactiae* group B, *Neisseria gonorrhoeae, Chlamydia trachomatis, Mycoplasma hominis,* and *Ureaplasma urealyticum.*

9. Use according to any one of claims 1 to 5 wherein the systemic bacterial infections are digestive system and abdominal infections caused by methicillin-susceptible *Staphylococcus epidermidis* (MSSE), methicillin-resistant *Staphylococcus epidermidis* (MRSE), *Streptococcus pyogenes,* Group G *Streptococci, Helicobacter pylori, Bacteroides fragilis, Clostridium perfringens, Escherichia coli,* quinolone-resistant *Escherichia coli, Salmonella spp., Shigella spp.,* ciprofloxacin-susceptible *Pseudomonas aeruginosa,* and *Clostridium difficile.*

10. Use according to any one of claims 1 to 5 wherein the systemic bacterial infections are bloodstream infections caused by methicillin-susceptible *Staphylococcus aureus* (MSSA), methicillin-resistant *Staphylococcus aureus* (MRSA) including ciprofloxacin-resistant strains, methicillin-susceptible *Staphylococcus epidermidis* (MSSE), methicillin-resistant *Staphylococcus epidermidis* (MRSE), *Streptococcus pyogenes,* penicillin-resistant *Streptococcus pneumoniae,* Beta-lactamase positive *Haemophilus influenzae, Legionella pneumophila,* and *Escherichia coli.*

11. Use according to any one of claims 1 to 5 wherein the systemic bacterial infections are bone and joint infections caused by methicillin-susceptible *Staphylococcus aureus* (MSSA), methicillin-resistant *Staphylococcus aureus* (MRSA) including ciprofloxacin-resistant strains, Group G *Streptococci,* and penicillin-resistant *Streptococcus pneumoniae.*

12. Use according to any one of claims 1 to 5 wherein the systemic bacterial infections are central nervous system infections caused by methicillin-susceptible *Staphylococcus aureus* (MSSA), methicillin-resistant *Staphylococcus aureus* (MRSA) including ciprofloxacin-resistant strains, methicillin-susceptible *Staphylococcus epidermidis* (MSSE), methicillin-resistant *Staphylococcus epidermidis* (MRSE), penicillin-resistant *Streptococcus pneumoniae,* Beta-lactamase positive *Haemophilus influenzae, Listeria monocytogenes,* and *Neisseria meningitidis.*

13. The use as claimed in any one of the preceding claims wherein said mammal is a human.

14. A tablet comprising an amount of 25 to 1200mg of ozenoxacin, and a suitable carrier.

15. A tablet according to claim 14 wherein the amount of ozenoxacin is 50 to 800mg.

16. A tablet according to claim 14 or 15 wherein the suitable carrier comprises a mixture of:
(i) a water soluble inert filler selected from mannitol, xylitol, sucrose, lactose compounds, maltodextrin, dextran, dextrin, modified starches, dextrose, sorbitol, and dextrates, and mixtures thereof, in a proportion of 5 to 70% in relation to the total weight of the tablet;
(ii) a disintegrant selected from pregelatinized starch, low-substituted hydroxypropyl cellulose, cross-linked sodium salt of a carboxymethyl ether of starch, cross-linked sodium carboxymethyl cellulose, cross-linked polyvinylpyrrolidone, sodium starch glycolate, sorbitan fatty acid esters, docusate sodium, polyoxyethylene sorbitan fatty acid esters, and microcrystalline cellulose, and mixtures thereof, in a proportion of 2 to 30% in relation to the total weight of the tablet;
(iii) a lubricant selected from magnesium stearate, calcium stearate, glyceryl monostearate, glyceryl palmitostearate, hydrogenated castor oil, colloidal silicon dioxide, glycerin, hydrogenated vegetable oil, mineral oil, polyethylene glycol, sodium benzoate, sodium lauryl sulfate, sodium stearyl fumarate, stearic acid, talc, and zinc stearate, and mixtures thereof, in a proportion of 0.2 to 10% in relation to the total weight of the tablet; and
(iv) a direct compression excipient selected from calcium hydrogen phosphate hydrates, spray-dried maltose, starch acetates, poly-lactic acid, and microcrystalline cellulose, and mixtures thereof, in a proportion of 5 to 70% in relation to the total weight of the tablet.

17. A tablet according to claim 16 wherein
(i) the water soluble inert filler is mannitol in a proportion of 8 to 30% in relation to the total weight of the tablet;
(ii) the disintegrant is a mixture of pregelatinized starch in a proportion of 3 to 10% in relation to the total weight of the tablet, and low-substituted hydroxypropyl cellulose in a proportion of 3 to 10% in relation to the total weight of the tablet;
(iii) the lubricant is sodium stearyl fumarate in a proportion of 0.5 to 5% in relation to the total weight of the tablet; and
(iv) the direct compression excipient is calcium hydrogen phosphate dihydrate in a proportion of 8 to 30% in relation to the total weight of the tablet.

18. A tablet according to claim 17 wherein
(i) the proportion of mannitol is from 10 to 20% in relation to the total weight of the tablet;
(iia) the proportion of pregelatinized starch is from 3 to 7% in relation to the total weight of the tablet;
(iib) the proportion of low-substituted hydroxypropyl cellulose is from 3 to 7% in relation to the total weight of the tablet;
(iii) the proportion of sodium stearyl fumarate is from 1 to 3% in relation to the total weight of the tablet; and
(iv) the proportion of calcium hydrogen phosphate dihydrate is from 10 to 25% in relation to the total weight of the tablet.

19. A solid oral dosage form comprising an amount of 25 to 1200 mg of ozenoxacin, and a suitable carrier.
